Europäisches Patentamt

(19) European Patent Office

Office européen des brevets

(11) Publication number: **0 108 629**
A2

(12) **EUROPEAN PATENT APPLICATION**

(21) Application number: 83306745.7

(22) Date of filing: 04.11.83

(51) Int. Cl.³: **G 01 N 27/22**
**G 05 D 22/02**

(30) Priority: 04.11.82 US 439193

(43) Date of publication of application:
16.05.84 Bulletin 84/20

(84) Designated Contracting States:
DE FR GB IT

(71) Applicant: AXIOMATICS CORPORATION

Sudbury Massachusetts(US)

(72) Inventor: Suh, Nam P.
34 Maynard Farm Road
Sudbury Massachusetts 01776(US)

(72) Inventor: Waldman, Francis A.
5 Davis Road
Belmont Massachusetts 02178(US)

(72) Inventor: Wesphal, William B.
1 Orchard Circle
Marblehead Massachusetts 01945(US)

(72) Inventor: Yeo, Woon C.
3-4 Arizona Terrace
Arlington Massachusetts 02174(US)

(72) Inventor: von Turkovich, Richard
5 Davis Road
Belmont Massachusetts 02178(US)

(74) Representative: Smith, Philip Antony et al,
REDDIE & GROSE 16 Theobalds Road
London WC1X 8PL(GB)

(54) Moisture measurement device.

(57) The moisture content of pellets of polymer for moulding is controlled by comparison of its dielectric loss with that of a reference sample. A hopper through which the pellets pass (11,12) contains sensor(s) 13A and 13B each containing a sealed reference sample of known moisture content which is kept at the same temperature as that of the material flowing through the buffer. The sample container is annular and disposed between two annular plates to define capacitors which are connected in a bridge circuit 14. The pellets flow through the gaps between the annular plates and the container.

A control circuit 15 controls a heating system 10A to increase or decrease the drying of the pellets flowing through the hopper.

FIG.1

-1-

## MOISTURE MEASUREMENT DEVICE

### Introduction

This invention relates generally to methods for measuring the moisture content of a material and, more particularly, to a method for measuring such moisture content in dielectric materials, such as polymers, which method can be used in on-line processing of such materials and can compensate for temperature changes of the materials in the processing equipment.

### Background of the Invention

In general two basic techniques for moisture measurements have been primarily used in the art in various batch and on-line processing systems which attempt to provide accurate moisture measurements at low moisture contents. Such approaches utilize either extraction-of-water techniques (i.e. techniques which use dessication, electrolytic or titration processes) or techniques which determine some property of materials which is dependent on the moisture therein (i.e. its conductivity, dielectric constant, microwave or infrared absorption properties, etc.). Of such approaches only those based on the properties of materials which are dependent on the moisture therein appear to be readily adaptable for use in order to measure moisture content on a continuous or on-line basis.

In molding processes, particularly where plastic materials such as polymers are involved, the initial moisture content of the polymer material prior to the molding step may range from relatively wet to already sufficiently dry. When certain plastics, such as nylon and PET, are processed in a relatively wet state, the molecular weight of the materials decreases resulting in a substantial reduction in mechanical properties.

In many systems, therefore, a batch of polymer material is pre-processed by appropriately drying it to a desired state and then performing the required molding process. Such systems do not monitor any changes in the moisture content which may occur during processing over a period of time. Such approach often leaves the material in an overdried state in addition to the fact that a substantial amount of energy may be wasted during the pre-processing drying phase. A potentially damaging consequence of overdrying lies in the increase in the viscosity of the plastic material which occurs as a result. Since changes in viscosity increase the injection pressure during molding and affect all the other molding processing variables, it becomes difficult to control the molding process and, consequently, to control the dimensions of the subsequently molded part.

Accordingly, it is desirable that a measurement or monitoring of the changes in moisture content of the material be continuously performed during the molding process on an on-line basis and that the heating (drying) of the material be controlled so that the moisture content can be maintained within acceptable limits.

## Discussions of the Prior Art

Techniques which have been suggested for measuring moisture content for such control purposes and which are based on some property of the material which is dependent on its moisture content include the use of a microwave absorption technique as exemplified by the system sold by Kay-Ray Inc. of Arlington Heights, Illinois under the designation of Model 8100 and Model 8500 Moisture Systems. The primary disadvantage of using microwaves is that such techniques cannot detect the moisture content to a sufficient degree of accuracy which is required in many applications. Such systems, for example, can measure moisture content to within 0.3% which accuracy is not sufficient for most hygroscopic resins, at the specified moisture content levels for use in on-line processing, where accuracies often must be within a range of 0.02% - 0.05%.

Another approach which has been suggested is to use infrared absorption techniques such as those utilized in systems sold by Anacon Corp. of Brighton, Massachusetts under the designation Model 1106 and in systems sold by Moisture Systems Corp. of Hopkington, Massachusetts under the designation Quadra-Beam Model 475.

A major problem with infrared absorption approaches is that such techniques measure substantially only the surface moisture content of a material which is being processed due to the rapid attenuation of infrared electromagnetic waves in these materials. At moisture content levels utilized in most process applications, however, most of the water in the material is bound within the polymer material itself and the water molecule concentration in the material is not uniform. Since infrared energy does not penetrate much below the surface of the material such technique cannot detect such bound-water molecules and does not provide an accurate measure of the moisture content of the overall material.

Another approach has been to monitor the dielectric constant of the material during the processing, as utilized in systems sold by Moisture Control Systems, Inc. of Vincennes, Indiana under the Model Designation MCS401 and by Forte Technology, Inc. of Canton, Massachusetts under the designation Model 4000. It is found, however, that the dielectric constant of materials is not sufficiently sensitive to moisture at low moisture levels to make such approaches of much use when the material being used has a relatively low moisture content.

A further approach has been suggested by U.S. Patent 4,320,599 issued to Suh et al. in which the system described therein measures changes in the dielectric loss factor, sometimes referred to as the dielectric loss tangent (the dielectric loss tangent is in effect the dielectric loss factor divided by the dielectric constant). The moisture content of a material affects the dielectric loss and the dielectric loss in turn determines changes in the phase angle of the impedance of the material (dielectric loss is, in effect, the imaginery part of the impedance). Such an approach can be contrasted with previous techniques which measure changes in capacitance of the material which are determined by changes in the dielectric constant of the material.

The particular system described in detail in such patent is primarily directed to the use of such measurement technique on a sample, or batch, of material rather than on a continuing, or on-line, basis. The patent does discuss the possibility of providing on-line measurements utilizing comparative measurements of the dielectric loss factor. However, since the dielectric loss is relatively sensitive to temperature variations, if such an approach is to be used in an on-line system, some technique for compensating for temperature variations must be devised. Although the Suh et al. patent discusses such a need, the only approach suggested therein is to provide for continuous absolute temperature measurements and then to use a microprocessor for compensating for the effects of temperature changes as they occur. No specific system or method for doing so beyond the mere suggestion of the use of microprocessor techniques is disclosed in the patent. Unfortunately, the use of such microprocessor techniques appears to require a system which not only is relatively complicated in design and operation but one which becomes relatively costly to produce.

It is desirable, therefore, that a method be devised for providing continuous on-line moisture content determinations which automatically compensate for temperature variations in an easier and less costly manner without the need for microprocessor techniques and which is sufficiently accurate to provide a reliable moisture determination for controlling the moisture content of a material in many applications.

## Summary of the Invention

A method, and a system for implementing such method, in accordance with the invention utilizes a continuous moisture measurement technique in which the moisture content of a material which is to be used in a molding process, for example, is continuously compared to that of a standard or reference sample of such material having a known moisture content. The sensor device utilized in a particular embodiment of the system for such purpose is designed effectively in the form of a coaxial capacitor element, arranged to include the sample standard in a manner such that the material to be processed, which normally may be in the form of pellets, for example, continuously flows through the sensor.

The sensor is designed so that the temperature of the standard sample material is maintained at the same bulk temperature as that of the material which is to be processed, the standard sample being hermetically sealed in order to prevent any changes in its known moisture content. Thus, the temperature responses of the material being measured and the standard sample are effectively matched and changes in the moisture content of the measured material can be compared accurately to that of the known standard using impedance comparison techniques based on the dielectric loss factor to produce a determination thereof substantially independently of temperature variations. Such moisture content variations can further be used, for example, to

34523

-7-

control, on-line, the drying (heating) of the continuously flowing material to maintain its moisture content within acceptable limits in a processing system.

Moreoever, in a preferred embodiment of the invention in order to promote more rapid heat transfer within the hermetically sealed standard material, so that its temperature can be maintained the same as that of the continuously flowing material, the standard material is placed in a helium gas environment.

## Description of the Invention

The invention can be described in more detail with the help of the accompanying drawings wherein:

FIG. 1 shows in diagrammatic form a portion of a drying apparatus in which the moisture measuring system of the invention can be used;

FIG. 2 shows in more detail a cross-section view of a sensor device and standard reference cell which can be used in the system of FIG. 1;

FIG. 3 shows a partial block diagram and partial circuit diagram of a measurement and control system for measuring the moisture content of the material which is being processed in the system of FIG. 1 and for controlling the drying thereof; and

FIG. 4 shows exemplary qualitative curves showing moisture content in a material as a function of the distance along the drying apparatus of FIG. 1.

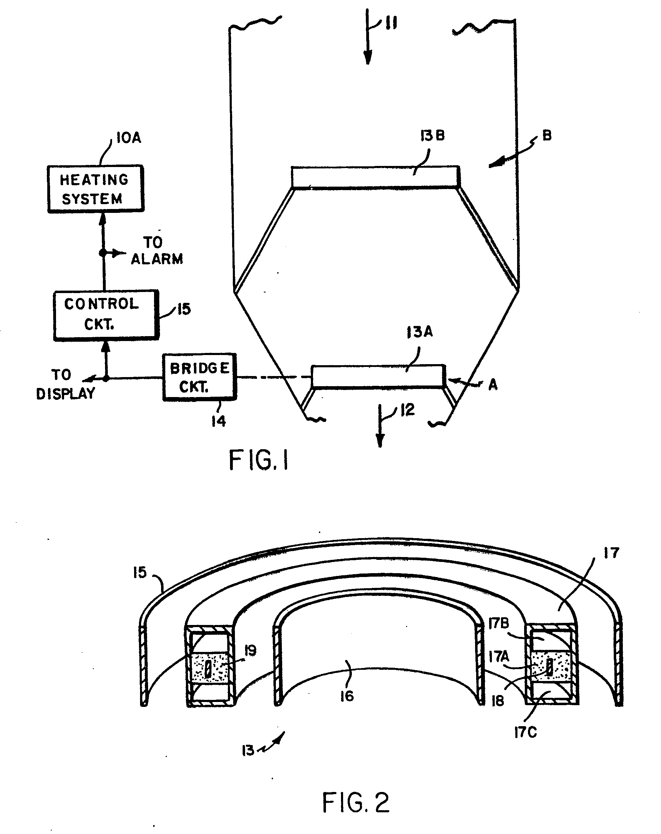

As can be seen in FIG. 1, a hopper 10 has a material in the form of pellets, for example, to be processed supplied at its

BAD ORIGINAL

-8-

upper end as shown by arrow 11. The hopper is suitably heated by heating system 10A to dry the pellets as they move through the hopper in their path to the exit thereof shown by arrow 12 from which point they are supplied to a suitable molding apparatus as would be well known in the art. The heating system can be of any known type used in the art for drying the pellets as they pass through the hopper 10 and need not be disclosed in further detail.

At least one sensor 13 is appropriately mounted within the hopper 10 at a selected position, e.g., adjacent the exit of hopper 10 as shown by sensor 13A or at a position higher up in the hopper as shown by sensor 13B, or at both positions, as discussed below. The sensor 13 includes a standard sample of the material to be processed, such sample having a known moisture content. The temperature of the standard sample is maintained at substantially the same temperature as the bulk temperature of the material as it flows through the sensor. The moisture content of the material at the sensor position is compared to that of the standard reference material in the sensor utilizing appropriate bridge circuitry 14 as discussed below.

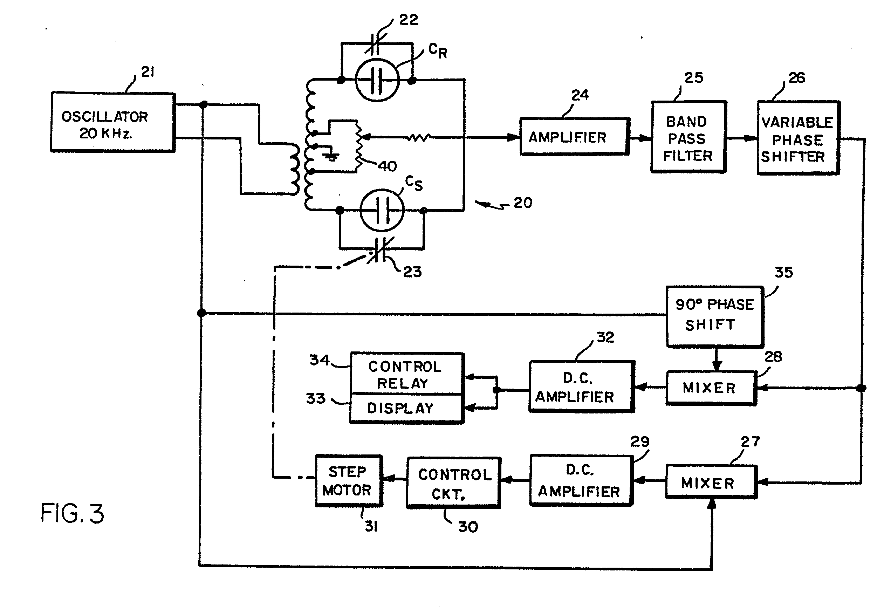

If the moisture content of the reference standard and of the flowing material to be processed are the same, the bridge is balanced and its output (e.g. effectively "zero") indicates, that the moisture content of the continuously flowing material is at the known value of that of the standard reference. If the moisture content of the flowing material to be processed is different from that of the refrence sample, the bridge circuit becomes unbalanced in one or the other direction, the unbalance thereof representing the change in the moisture content of the flowing material. The output of the bridge can be suitably displayed to show the changes in moisture content to the user. If the change in moisture content becomes sufficiently large as to exceed a predetermined level, in either direction, appropriate control circuitry 14A can be utilized either to shut off the heating system, to actuate an alarm, or to otherwise control the heat flow so as to bring the moisture content to within a desired range. Such bridge measurement and control system is discussed in more detail below with reference to FIG. 3.

FIG. 2 shows a view in section of a particular embodiment of a sensor 13. As can be seen therein, the sensor is in the form of a coaxial structure having an outer cylindrical coaxial element 15 and an inner cylindrical coaxial element 16. Positioned between such elements is a standard reference cell structure 17 having a central compartment 17A which includes an electrode element 18 centrally mounted therein in a suitable manner. Compartment 17A is filled with pellets of the material which is to be processed and is referred to as a standard reference sample 19 of such material. Upper and lower compartments 17B and 17C form hollow extensions of compartment 17A so that the outer surface areas of the side walls of cell 17 opposite elements 15 and 16 generally correspond to the inner surface areas of the latter elements.

The elements of sensor 13 shown in FIG. 2 form capacitances which correspond to capacitors shown in the bridge measurement and control circuit diagram of FIG. 3, as discussed below. For example, the element 16 and the inner wall of reference cell 17 form a first capacitance in parallel with a further capacitance formed by element 15 and the outer wall of element 17. The combination of such capacitances can be viewed as forming a single sensor capacitance $C_S$. The electrode 18 in reference cell 17 also forms a pair of parallel capacitances, one between electrode 18 and the inner side wall of element 17 and one between electrode 18 and the outer side wall thereof. Such combination of capacitances can also be viewed as forming a single reference capacitance $C_R$. The pellets of the material to be processed flow through the opening between reference cell 17 and the outer and inner coaxial elements 15 and 16.

As can be seen in FIG. 3, bridge measurement and control circuitry for use with the sensor of FIG. 2. utilizes an AC bridge circuit 20 which has an AC excitation obtained from oscillator 21. In a particular example discussed herein in which the material being pre-processed for use in a mold is a nylon material, the oscillator is found to be most effective if it provides an AC signal having a frequency of about 20 KHz. One arm of the bridge circuit includes a capacitance $C_R$ which represents the capacitance of the reference cell 17 of FIG. 2 as discussed above. An opposite arm thereof includes a capacitance $C_S$ which represents the capacitance of the sensor as also discussed above with reference to FIG. 2. A relatively small trim capacitor 22 is utilized in parallel with the reference capacitance $C_R$ for purposes discussed below. A variable capacitor 23, the value of which is controlled by a feedback path, discussed below, is in parallel with the sensor capacitor $C_s$.

The output of the bridge circuit is supplied to amplifier 24 the amplifier output in turn being filtered by a suitable band pass filter 25. The filtered output is supplied to a variable phase shifter 26 which produces an output for supply to a first in-phase mixer 27 and a second quadrature mixer 28. Mixer 27 mixes the output from variable phase shifter 26 with the in phase oscillator signal from oscillator 21, the mixer output being supplied to a DC amplifier 29 which is in turn supplied to a stepper motor control circuit 30, the level of the DC amplifier causing the stepping motor 31 to step to a particular position which changes the value of variable capacitor 23 (the value of which is arranged to be changed in discreet steps) until the bridge is rebalanced. The purpose thereof is discussed below.

The output of phase shifter 26 is also mixed with the quadrature output from oscillator 21. The output of mixer 28 is supplied to DC amplifier 32, changes in the voltage output of which effectively represent changes in the dielectric loss (i.e., changes in the moisture content) of the continuously flowing material which passes through sensor 13. Such voltage can be suitably displayed visually by display means 33, e.g. an appropriate plotter, cathode ray tube, digital display device, etc., as would be well known to the art. If the output of DC amplifier 32 exceeds a particular level or falls below a particular level (i.e., the bridge circuit becomes unbalanced in either direction), such levels being selected as the limits of a desired range of moisture content for the particular material being tested, the DC amplifier output can be used to actuate appropriate relays for providing an audible alarm, for example, to inform the operator that the moisture content is either too high or too low for the particular molding operation in which the material is to be used. Such limits can be set empirically depending on the specific material being used and also depending on where the sensor is placed. The user can then shut off the system so that the heating system can be appropriately manipulated to increase or decrease the heating effect on the material (depending on whether the material is too moist or not moist enough). Alternatively the heating system can be directly controlled to automatically increase or decrease the heating, or drying, of the material.

Thus, if a single sensor is placed at the exit (location A) in FIG 1, i.e., essentially at the point where the material enters the mold, the limits are set at predetermined levels in accordance with the desired limits normally prescribed by the mateial manufacturer, for example, as representing the allowable range of moisture content levels for molding purposes. Alternatively such limits can be predetermined empirically.

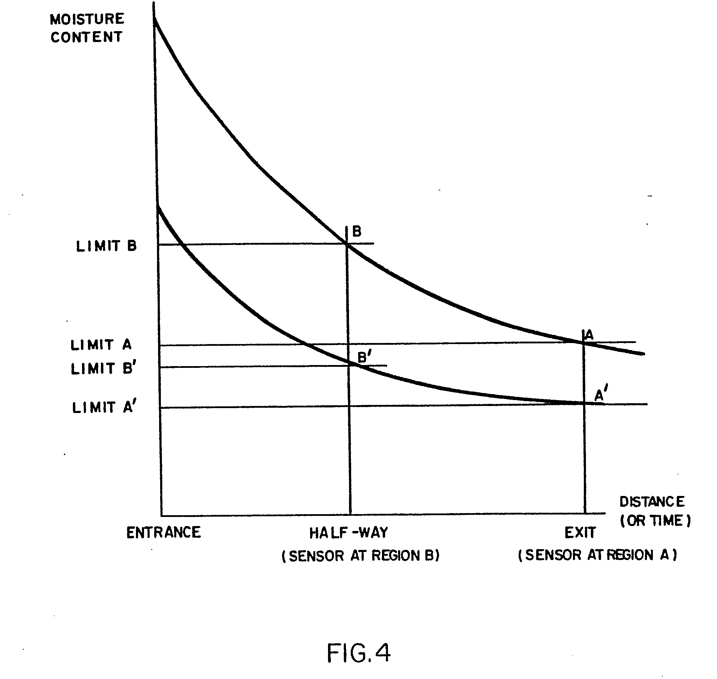

Thus, FIG. 4 illustrates exemplary curves which show qualitatively the moisture content changes which occur along the hopper in order to reach the desired limit levels at the exit thereof (at position A). The limits A and A' represent the maximum and minimum alarm levels required (equivalent to maximum

34523

-13-

desired moisture content and minimum desired moisture content).
The quantitative levels in a particular case will depend on the
material being used, the rate of its movement in the hopper, the
humidity of the air, the drying temperature, etc. and once such
factors are known the curves can be empirically generated as
follows, for example.

The drying temperature is first arranged so that pellets
of material at the exit of the hopper have a predetermined
moisture content A (a sample of the material at such point can be
measured as by using the technique of the above mentioned Suh et
al. patent, for example, so as to arrive at the desired
predetermined level at A). Samples of material can then be
removed at other various hopper distances and their moisture
content determined (also using techniques of the above Suh et al.
patent, for example) so that a curve of moisture content vs.
hopper distance is generated. A similar curve can be generated
by arranging the system to provide material having a
predetermined moisture content A' at the exit. With a knowledge
of such curves and the conditions under which they were
generated, many different control schemes would be possible
depending upon the number of sensors and their locations, as
explained below.

Thus, if a single sensor is used at exit location A, the
moisture content at such location is monitored as discussed above
with reference to FIG. 3 and if such content exceeds the upper or
lower limits, the operator can be alerted (as by alarms as
discussed above) so that corrective action can be taken.

34523

-14-

In some applications it may be desirable to arrange the system so that corrective action can be taken before the moisture content of the material at the exit reaches the predetermined limits. In such a case a second sensor can be placed at a suitable position in the hopper, e.g. at the half-way point at location B. The predetermined limits at such location are then shown in the exemplary (and previously generated) curves of FIG. 4 at B and B'. The second sensor can be used (with another bridge circuit similar to that shown in FIG. 3) to alert the operator when such latter limits have been exceeded at the half-way point (even though not yet exceeded at the exit) so that the operator can take corrective action at an earlier point in time than was possible when only a single sensor is used at exit A.

In order to initialize the bridge circuit and associated circuitry of FIG. 3, the following procedure can be used to set up the bridge circuit for the above discussed operation.

First of all, the reference cell is filled with reference material which is completely dry and the hopper particularly in the vicinity of the sensor, is also filled with the same dry material. With material having the same moisture content (in this case effectively zero percent) the bridge resistor 40 is set to balance the bridge output to zero (in effect, to set the output of DC amplifer 32 to zero) as shown by a suitable display device 33. Such initial balancing zeros out any off-sets due to the circuitry.

Next, pellets having a known moisture content are placed

-14-

-15-

in the sensor (the dry pellets remaining in the reference cell). For example, if the system is to be used to determine changes in the moisture content of nylon pellets, pellets having a known moisture content of 0.15% are utilized. Such moisture content can be determined by using known "batch" measuring techniques, e.g., the technique disclosed in the aforesaid Suh et al. patent.

The above known moisture content is selected to be approximately in the center of a useful range of moisture content in a particular application. Thus, in the case of nylon material, the useful range of moisture content in a molding apparatus lies within a range from 0.1% to 0.2%, such range being pre-determined empirically by the user to assure acceptable molding characteristics or such range being obtained from the mold manufacturer, for example, as discussed above.

Pellets having the selected known moisture content are placed in the sensor and the off-set voltage produced thereby is then noted. The bridge is then balanced (by adjusting resistor 40) to bring the output thereof to zero. Therefore, so long as pellets having the known moisture content of 0.15% are flowing through the sensor, the bridge output is zero and the moisture content is within the desired range (effectively at the center of the range).

When the moisture content is above or below such known level the bridge output will be balanced in one or the other direction and the output voltage level will be proportional to the change from such known moisture content.

In the system of FIG. 3, the bridge output is supplied to the amplifier 24 and the remaining circuitry shown therein to produce a DC amplified voltage output at DC amplifier 32 which represents the change in the moisture content relative to the known moisture content of the reference sample material. The gain of DC amplifer 32 can be appropriately set so that the voltage output is at a level effective for display by a suitable display device.

Control relays 34 can be arranged to be actuated when the DC voltage output from amplifier 32 is equal to or exceeds either of the values (i.e., in either direction) which correspond to the selected upper and lower limit valves of the moisture content. Thus, for a nylon material, a pair of relays are used and are arranged to be actuated, respectively, by the value of the DC output voltage from DC amplifier 32 corresponding to a moisture content at the lower limit of 0.1% and at the upper limit of 0.2%. Such relays may be used to actuate an audible alarm, for example, to alert the operator that the limits have been reached so that the operator can shut down the system to permit remedial action to be taken. For example, if the pellets are too moist the operator may stop or slow down the flow thereof to permit the pellets to be subject to the drying cycle for a longer period of time so that they become sufficiently dry and their moisture content once again falls within the acceptable limits. On the other hand if the pellets are too dry the operator may shut down the drying system entirely until the moisture content falls within the acceptable limits.

In order to assure that determination of the moisture content of the flowing material is effectively independent of temperature changes in the material, the configuration of sensor 13 is arranged so that the bulk temperature of the reference sample and that of the continually flowing pellets being sensed are substantially the same. Thus, in the configuration shown in FIG. 2, the reference sample tends to assume the same bulk temperature as the flowing material because of the heat transfer which occurs from the flowing pellets through the reference cell walls to the reference sample. In some applications in order to promote a fast transfer of heat (especially where the temperature of the material in the hopper changes relatively rapidly) so that changes in the bulk temperature of the flowing material are rapidly reflected in the bulk temperature of the reference sample (i.e. the temperature of the latter follows that of the former with little or no time lag), helium gas can be used in the hermetically sealed reference sample compartment 17A. It is found that the use of helium gas therein effectively enhances the heat transfer so that the temperatures of the reference sample and the flowing material remain relatively closely matched during processing. In some systems where temperature of the material in the hopper changes relatively slowly, gases other then helium (e.g., air) can be used.

While the above discussion discloses a particular embodiment of the invention, other embodiments will occur to those in the art. Thus, for some materials a different excitation frequency may be selected as more appropriate for use and suitable changes in the circuitry and/or sensor arrangements

-18-

may be desirable or required for best operation at such frequency. Other modifications may occur in the art within the spirit and scope of the invention and, hence, the invention is not to be construed as limited to the particular embodiment described herein except as defined by the appended claims.

-19-

CLAIMS:

1. A method of monitoring the moisture content of a material comprising the steps of

supplying a continuous flow of said material;

placing a reference sample of said material having a known moisture content in the path of said continuous flow of material;

maintaining the bulk temperature of said reference sample at substantially the same bulk temperature as that of said continuous flow of material; and

determining changes in the dielectric loss of said continuous flow of material relative to the dielectric loss of said reference sample, said changes in the dielectric loss of said material representing changes in the moisture content thereof.

2. A method in accordance with claim 1 wherein said material is a polymer plastic material.

3. A method in accordance with claim 1 wherein said material is in the form of pellets.

4. A method in accordance with claim 1 and further including the step of providing an indication of the changes in the dielectric loss of said material.

5. A method in accordance with claim 4 wherein said last-named step includes providing a visual indication of said changes.

6. A method in accordance with claim 4 wherein said last-named step includes providing an audible indication of said changes.

7.    A method in accordance with claim 1 and further including the steps of

drying said continuous flow of material; and

controlling the drying of said continuous flow of material in response to said changes in the dielectric loss thereof.

8.    A method in accordance with claim 7 wherein said controlling step includes the step of maintaining the dielectric loss of said material within preselected limits.

9.    A method in accordance with claim 1 and further including the step of supplying said continuous flow of material to a processing system and wherein changes in the dielectric loss of said material are determined substantially at the point where said material is supplied to said processing system.

10.    A method in accordance with claim 1 and further including the step of maintaining the moisture content of said reference sample at said known moisture content level.

11.    A method in accordance with claim 10 wherein the moisture content of said reference sample is maintained at substantially zero percent.

12.    A system for monitoring the moisture content of a material comprising

means for supplying a continuous flow of said material;

sensor means being positioned in the path of said continuous flow of material and including

reference means containing a reference sample of said material having a known moisture content, said reference means being arranged in said sensor means in the path of said continuous flow of material so that the bulk temperature of said reference sample is maintained at substantially the same temperature as that of said continuous flow of material; and

- 20 -

0108629

means responsive to changes in the dielectric loss of said continuous flow of material relative to the dielectric loss of said reference sample for providing an output representing changes in the moisture content of said continuous flow of material.

13. A system in accordance with claim 12 wherein said sensor means has a configuration which forms a sensor capacitance, a selected portion of the impedance of which represents the dielectric loss of said continuous flow of material, said dielectric loss being dependent on the moisture content thereof;

said reference means having a configuration which forms a reference capacitance, a selected portion of the impedance of which represents the dielectric loss of said reference sample, said dielectric loss being dependent on the moisture content of said reference sample.

14. A system in accordance with claim 13 and further wherein said determining means includes means for comparing the selected portions of the impedance of said sensor capacitance and said reference capacitance to provide an output signal representing said changes in the dielectric loss of said continuous flow of material relative to that of said reference sample.

15. A system in accordance with claim 14 wherein said comparing means includes an A-C bridge circuit, said impedances forming arms of said bridge circuit.

16. A system in accordance with claim 15 wherein said bridge circuit includes A-C excitation means having a preselected frequency.

17.  A system in accordance with claim 16 wherein said continuous flow of material is a polymer plastic material.

18.  A system in accordance with claim 17 wherein said polymer plastic material is nylon and the frequency of said A-C excitation signal is appropriately 20 kHz.

19.  A system in accordance with claim 13 wherein said sensor means comprises

a pair of coaxial elements, said reference means being positioned between said coaxial elements so as to form with said coaxial elements said sensor capacitance;

said reference means having a conductive element positioned therein so as to form with said reference means said reference capacitance.

20.  A system in accordance with claim 13 and further including means responsive to said output for visually indicating the changes in said moisture content.

21.  A system in accordance with claim 13 and further including means responsive to said output for audibly indicating when the changes in said moisture content exceed preselected limits.

22.  A system in accordance with claim 13 and further including

means for drying said continuous flow of material; and

means responsive to said output for controlling the operation of said drying means to maintain the changes in the moisture content of said continuous flow of material within preselected limits.

23.  A system in accordance with claim 12 and further including means for compensating for changes in the dielectric constant  of said continuous flow of material.

24. A system in accordance with claim 12 wherein said reference sample is contained in said reference means in a selected gas environment.

25. A system in accordance with claim 24 wherein said selected gas environment is helium.

26. A system in accordance with claim 12 wherein said continuous flow of material supplying means has an entrance and an exit and said sensor means is positioned substantially at the exit thereof.

27. A system in accordance with claim 12 wherein said continuous flow of material supplying means has an entrance and an exit and said sensor means is positioned between the entrance and the exit thereof.

28. A system in accordance with claim 27 wherein said sensor means is positioned substantially half-way between the entrance and exit thereof.

29. A system in accordance with claim 12 wherein said continuous flow of material supplying means has an entrance and an exit and said sensor means includes a first sensor positioned substantially at the exit and a second sensor positioned between the entrance and the exit.

1/3

FIG.1

FIG.2

FIG. 3

3/3

FIG.4